# EUROPEAN PATENT SPECIFICATION

(11) **EP 0 451 221 B1**
(45) Date of publication and mention of the grant of the patent: **12.10.1994**
(21) Application number: 90909257.9
(22) Date of filing: 05.06.1990
(51) Int. Cl.: A61K 37/62, C07H 17/00, C07H 15/12, A61K 31/70

(54) **CHIMERIC DNA-RNA CATALYTIC SEQUENCES**
CHIMERE DNS-RNS KATALYTISCHE SEQUENZEN
SEQUENCES CATALYTIQUES CHIMERIQUES D'ADN/ARN

(30) Priority: 31.08.1989 US 401613
(43) Date of publication of application: 16.10.1991
(73) Proprietor: CITY OF HOPE, Duarte California 91010 (US)
(72) Inventor: ROSSI, John, J., Glendora, CA 91740 (US); CHANG, Pairoj, San Dimas, CA 91773 (US); KAPLAN, Bruce, E., Claremont, CA 91711 (US)
(74) Representative: Marchant, James Ian
(86) International application number: US9003102
(87) International publication number: WO9103162

(56) References cited:
- Chem. Abstract, vol 112, no. 7, issued 12 Feb 1990 (Columbus, Ohio, USA) W. Gerlach, et al., "Synthetic Ribozymes for in ViVo Inactivation of Prokaryotic or RNA Transcripts", see pages 336-337, col 2, seethe abstract no. 51284j, Eur. Pat. Appl. no. EP 321,201 21 June 1989.
- Chemical Abstract, vol., 112, no. 19, issued 07 May 1990 (Columbus, Ohio, USA) N. Sarver, et al, "Ribozymes as Potential Anti-HIV-1 Therapeutic Agents", see page 420, col. 2, see the abstract no. 17548q, Science, 1990, 247 (4947), 1222-5(Eng).
- Chem Abst. , vol., 112, no.7, issued 12 Feb. 1990 (Columbus, Ohio, USA) M Cotton, et al., Ribozyme Mediated Destruction of RNA in ViVo", see page 501, col. 1, see the abst. no. 52942j, EMBO J, 1990, 8(12), 3861-6 (Eng).
- Chem. Abst. vol. 110, no. 21, issued 22 May 1989, (Columbus, Ohio, USA) T. R. Cech et al., RNA Ribozyme Polymerases, Dephososphorylases, Restriction Endoribonucleases and Methods for Their Manufacture", see page 226, col. 2, see the abst. no. 187321K, PCT Int. PPl. W08804,300 16 June 1988.
- Nature, vol. 344, isued 05 April 1990, J. Peneault, et al., Mixed Deoxyribo - and Ribooligonucleotides with Catalytic activity see pages 565-567.
- Proceeding of the National Academy of Sciences, Vol. 86, no. 23, issued Dec. 1989 (USA) F. H. Cameron, et al., "Specific Gene Suppression by Engineered Ribozymes in Monkey Cells", see pages 9139-9143.

## Description

This invention pertains to DNA-RNA catalytic molecules. More particularly the invention pertains to chimeric DNA-RNA-DNA-RNA-DNA catalytic molecules effective to cleave HIV-1 RNA sequences, for example.

Ribozymes are structural RNA molecules which mediate a number of RNA self-cleavage reactions. Two distinct trans-acting ribozymes, "hammerhead" and "hairpin," having different secondary structures have been identified. Oncogenes and Aids (1990) [citation] states:
"Another possible synthetic approach is the development of a chimeric molecule containing a ribonucleotide catalytic center and deoxyribonucleotide flanking sequences. It is also conceivable that chimeric catalysts comprised of an RNA catalytic center and DNA flanking sequences will retain biological activity while having greater stability."
Perreault, et al., Nature, 344:565-567 (1990), describes certain mixed deoxyribo and ribooligonucleotides with catalytic activity. No RNA-DNA catalytic molecules of practical therapeutic utility are known.

This invention provides chimeric DNA/RNA catalytic molecules useful to cleave RNA sequences. The invention specifically provides two different chimeric DNA-RNA-DNA-RNA-DNA catalytic molecules which are targeted to cleave HIV-1 RNA sequences. These chimeric molecules include DNA sequences which flank a catalytic RNA center. Interaction with the HIV-1 substrate RNAs is achieved by Watson-Crick base pairing of the DNA flanking sequences with HIV-1 RNA. The catalytic ribonucleotide center cleaves the phosphodiester bond of the substrate HIV-1 RNA at the expected location.

In general the catalytic molecules of the invention function as hammerhead or hairpin ribozymes. The preferred molecular construct consists of two known RNA catalytic sequences each flanked by a DNA sequence at the respective 3' and 5' termini and coupled by a DNA sequence at the corrseponding 5' and 3' termini. These molecules may accordingly be represented by the formulae I and II::
I. 3' X - AAAG - Y - AGUAGUC - Z 5'
or
II. 3' X - CAAAG - Y - AGUAGUC - Z 5'
in which X, Y and Z are DNA sequences and AAAG, CAAAG and AGUAGUC are catalytic RNA sequences.

The flanking X and Z components may be any DNA sequences that allow base pairing with the substrate RNA at appropriate positions adjacent to the substrate cleavage site. These flanking sequences may be phosphodiester, phosphorothioate, methyl phosphonate, methyl phosphate or similar moieties.

Y may be any DNA sequence that base pairs inter se in the manner required for catalytic cleavage of the substrate by the RNA sequences preferably as shown in base paired form in Formula III: 
III.  5′ C-G 3′
         A-T
         G-C
         G-C
        A   G
         G T
The catalytic molecules of this invention can be synthesized in known manner by commercially available DNA synthesizers such as those produced by Applied Biosystems or Milligen. See, e.g., Perreault, et al, supra.

The X and Z sequences may be substituted at the respective 3' and 5' ends with ligands to facilitate cell entry, targeting within the cell and ultimate stability of the catalysts. Such ligands include by way of example other nuclotides, proteins, carbohydrates, lipids, steroid hormones and cholesterol.

The catalytic molecules of the invention are administered by known and available delivery agents or systems, including liposomes, defective viral particles, viral capids, and standard DNA/RNA transfective procedures.

Figure 1 illustrates one catalytic molecule of the invention base paired to an HIV-1 sequence. The RNA portion of the molecule is encircled.

Figure 2 illustrates a second catalytic molecule of the invention base paired to another HIV-1 sequence. The RNA portion of the molecule is encircled.

Figure 3A depicts a ribonuclease A digestion of the catalytic molecule of Figure 1 as compared with an equivalent all DNA molecule. The conditions were 10 units of commercial (Sigma) pancreatic ribonuclease in 2XSSC buffer added to the oligonucleotides which were in 10 microliters of 50 mM Tric-HCl buffer (pH 8.0). The RNAse was incubated with the sample for 10 minutes before the ³²-P end labelled DRDRD or DNA molecules were electrophoresed in a 15% polyacrylamide gel containing 8M urea. The gel was autoradiographed for 10 minutes to get the exposure depicted.

Figure 3B depicts a cleavage reaction involving the catalytic molecule of Figure 1 under conditions described in Chang, et al., Clinical Biotechnology, 2:23-31 (1990).

### EXAMPLE I

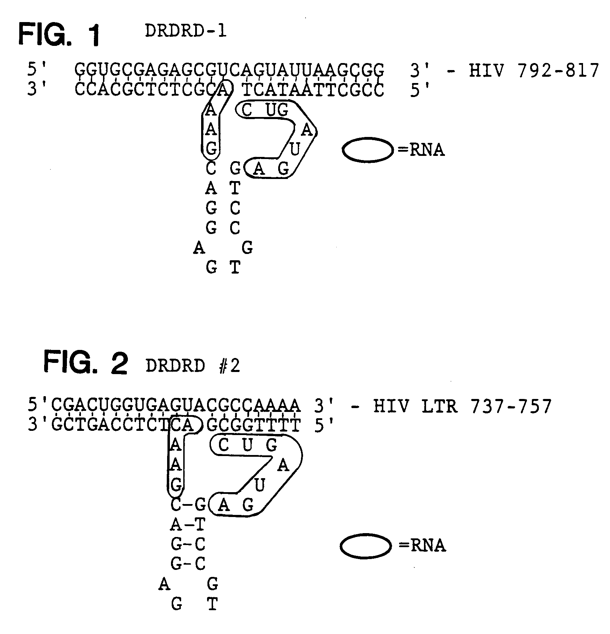

The catalytic molecule of Figure 1 was synthesized in known manner utilizing an automated oligonucleotide synthesizer manufactured by Applied Biosystems, Inc.

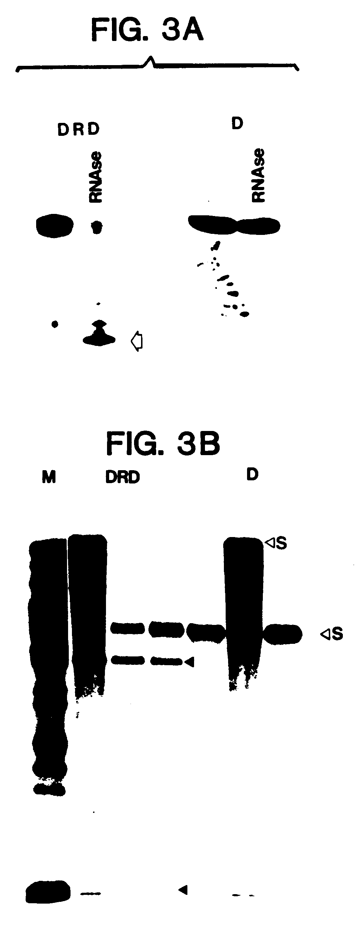

The result of ribonuclease A digestion of the catalytic molecule is shown by Figure 3A.

The catalytic molecule produced, as described, was used to cleave each of a 610 nuleotide long (S-610) and a 170 nucleotide long HIV-1 gag transcript. In brief, the buffer was 50 mM Tris-HCl, pH 7.5, 1mM EDTA, 10mM MgCl₂ at approximately 1 pmole of target, 3 pmole of ribozyme or DNA. The reactions were carried out at 37°C. for 12 hours. The substrate was either a 610 nucleotide long HIV-1 gag containing transcript (S-610) or a 172 nucleotide long HIV-1 gag containing transcript (S-172). The 5' cleavage product is indicated for both.

in Figure 3B the 5' cleavage product is shown for both transcripts. The 3' cleavage product for the 610 target is not visible due to poor reproduction of the autoradiograph, but is indicated in its position by a 3' P notation. As a negative control, an all DNA oligonucleotide (D) of the same sequence as the DRDRD molecule was incubated with the same substrates under the same conditions with the result that no cleavage was obtained.

Specific cleavage of an HIV-1 5' LTR splice site with a similar catalytic molecule has also been obtained.

## Claims

1. A catalytic molecule capable of cleaving an RNA sequence, the molecule comprising a DNA-RNA-DNA-RNA-DNA sequence wherein the RNA sequences are catalytic sequences; characterised in that the two outer DNA sequences are capable of base pairing with the RNA sequence to be cleaved and in that the central DNA sequence is any DNA sequence which base pairs *inter se* in the manner required for catalytic cleavage.

2. A catalytic molecule capable of cleaving an HIV-I RNA sequence at a known ribozyme cleavage site said molecule having the formula
3' X - AAAG - Y - AGUAGUC - Z 5'
or
3' X - CAAAG - Y - AGUAGUC - Z 5'
in which X and Z are DNA sequences that base pair with an RNA substrate at positions juxtaposed to said known cleavage site,
AAAG, CAAAG and AGUAGUC are RNA sequences,
Y is a DNA sequence that base pairs *inter se* in a manner required to permit said RNA sequences to cleave said substrate at said cleavage site.

3. A catalytic molecule having the following sequence: 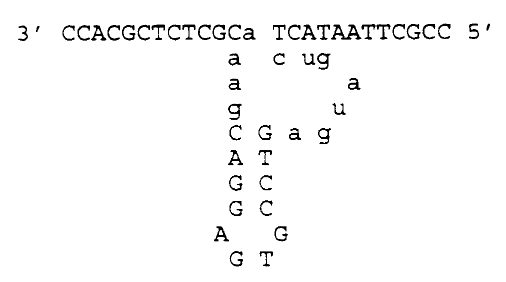 wherein lower case letters represent RNA.

4. A catalytic molecule having the following sequence: 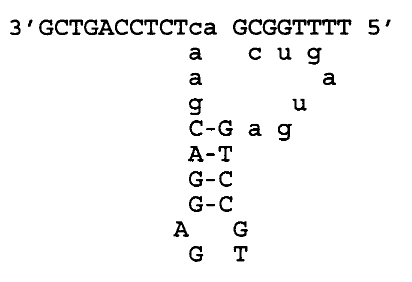 wherein the lower case letters represent RNA.

5. A catalytic molecule as claimed in any one of claims 1 to 4 in which the RNA sequence to be cleaved is an HIV-I sequence.

6. A catalytic molecule as claimed in claim 5 in which the HIV-I sequence comprises:
5' GGUGCGAGAGCGUCAGUAUUAAGCGG 3' - HIV 792-817.

7. A catalytic molecule as claimed in claim 5 in which the HIV-I sequence comprises:
5'CGACUGGUGAGUACGCCAAAA 3' - HIV LTR 737-757.

## Patentansprüche

1. Katalytisches Molekül, das zur Spaltung einer RNA-Sequenz imstande ist, welches Molekül eine DNA-RNA-DNA-RNA-DNA-Sequenz umfaßt, worin die RNA-Sequenzen katalytische Sequenzen sind; dadurch gekennzeichnet, daß die beiden äußeren DNA-Sequenzen zur Basenpaarung mit der zu spaltenden RNA-Sequenz imstande sind und daß die zentrale DNA-Sequenz irgendeine DNA-Sequenz ist, die inter se auf die zur katalytischen Spaltung erforderliche Weise basenpaart.

2. Katalytisches Molekül, das zur Spaltung einer HIV-I-RNA-Sequenz an einer bekannten Ribozymspaltungsstelle imstande ist, welches Molekül folgende Formel besitzt
3' X - AAAG - Y - AGUAGUC - Z 5'
oder
3' X - CAAAG - Y - AGUAGUC - Z 5'
worin X und Z DNA-Sequenzen sind, die mit einem RNA-Substrat an Positionen basenpaaren, die neben der bekannten Spaltungsstelle liegen,
AAAG, CAAAG und AGUAGUC RNA-Sequenzen sind,
Y eine DNA-Sequenz ist, die inter se auf eine erforderliche Weise basenpaart um zu ermöglichen, daß die RNA-Sequenzen das Substrat an der Spaltungsstelle spalten.

3. Katalytisches Molekül mit der folgenden Sequenz 
3' CCACGCTCTCGCa TCATAATTCGCC 5'
a  c ug
a        a
g       u
C G a·g
A T
G C
G C
A  G
G T
 wobei die Kleinbuchstaben RNA darstellen.

4. Katalytisches Molekül mit der folgenden Sequenz: 
3'GCTGACCTCTca GCGGTTTT 5'
a   c u g
a         a
g       u
C-G a g
A-T
G-C
G-C
A   G
G  T
 wobei die Kleinbuchstaben RNA darstellen.

5. Katalytisches Molekül nach einem der Ansprüche 1 bis 4, worin die zu spaltende RNA-Sequenz eine HIV-I-Sequenz ist.

6. Katalytisches Molekül nach Anspruch 5, worin die HIV-I-Sequenz folgendes umfaßt:
5' GGUGCGAGAGCGUCAGUAUUAAGCGG 3' - HIV 792-817.

7. Katalytisches Molekül nach Anspruch 5, worin die HIV-I-Sequenz folgendes umfaßt:
5'CGACUGGUGAGUACGCCAAAA 3' - HIV LTR 737-757.

## Revendications

1. Molécule catalytique capable de cliver une séquence d'ARN, molécule comprenant une séquence d'ADN-ARN-ADN-ARN-ADN dans laquelle les séquences d'ARN sont des séquences catalytiques, caractérisée en ce que les deux séquences d'ADN externes sont capables d'un appariement de bases avec la séquence d'ARN destinée à être clivée et en ce que la séquence d'ADN centrale est une séquence d'ADN quelconque donnant lieu à un appariement de bases *inter se* de manière requise pour le clivage catalytique.

2. Molécule catalytique capable de cliver une séquence d'ARN HIV-I au niveau d'un site de clivage connu de ribozyme, ladite molécule répondant à la formule
3' X - AAAG - Y - AGUAGUC - Z 5'
ou
3' X - CAAAG - Y - AGUAGUC - Z 5'
dans laquelle X et Z sont des séquences d'ADN qui donnent lieu à un appariement de bases avec un substrat d'ARN au niveau de positions juxtaposées audit site de clivage connu,
AAAG, CAAAG et AGUAGUC sont des séquences d'ARN,
Y est une séquence d'ADN qui donne lieu à un appariement de bases *inter se* de manière requise pour permettre auxdites séquences d'ARN de cliver ledit substrat audit site de clivage.

3. Molécule catalytique ayant la séquence suivante : 
3' CCACGCTCTCGCa TCATAATTCGCC 5'
a  c ug
a        a
g      u
C G a g
A T
G C
G C
A  G
G T
 dans laquelle les lettres en minuscule représentent l'ARN.

4. Molécule catalytique ayant la séquence suivante : 
3' GCTGACCTCTca GCGGTTTT 5'
a   c u g
a         a
g       u
C-G a g
A-T
G-C
G-C
A   G
G   T
 dans laquelle les lettres en minuscule représentent l'ARN.

5. Molécule catalytique telle que revendiquée dans l'une quelconque des revendications 1 à 4 dans laquelle la séquence d'ARN destinée à être clivée est une séquence de HIV-I.

6. Molécule catalytique telle que revendiquée dans la revendication 5 dans laquelle la séquence de HIV-I comprend:
5' GGUGCGAGAGCGUCAGUAUUAAGCGG 3' - HIV 792-817.

7. Molécule catalytique telle que revendiquée dans la revendication 5 dans laquelle la séquence de HIV-I comprend:
5'CGACUGGUGAGUACGCCAAAA 3' - HIV LTR 737-757.
